(19) Europäisches Patentamt
European Patent Office
Office européen des brevets

(11) EP 3 168 608 A1

(12) EUROPEAN PATENT APPLICATION

(43) Date of publication:
17.05.2017 Bulletin 2017/20

(51) Int Cl.:
$G01N\ 27/22$ [(2006.01)]    $G01N\ 33/12$ [(2006.01)]
$B65B\ 25/00$ [(2006.01)]    $G01N\ 33/02$ [(2006.01)]

(21) Application number: 15306815.0

(22) Date of filing: 16.11.2015

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicants:
• UNIVERSITE DE MONTPELLIER
34090 Montpellier (FR)
• CENTRE NATIONAL DE LA RECHERCHE
SCIENTIFIQUE
75794 Paris Cedex 16 (FR)

• Institut National de la Recherche Agronomique
(INRA)
75338 Paris Cedex 07 (FR)

(72) Inventors:
• GONTARD, Nathalie
34000 MONTPELLIER (FR)
• GUILLAUME, Carole
34380 MONTPELLIER (FR)
• SORLI, Brice
34530 MONTAGNAC (FR)

(74) Representative: Nony
11 rue Saint-Georges
75009 Paris (FR)

(54) **USE OF BIOPOLYMER IN A DIELECTRIC GAS SENSOR**

(57)    The present invention relates to the use of a biopolymer as a sensitive material in a dielectric gas sensor for water vapor, carbon dioxide, and/or ethanol.

PCB
Electrodes
Gluten layer
$14.4 \pm 2.3\ \mu m$
$17.4 \pm 5\ \mu m$

**Figure 1**

EP 3 168 608 A1

**Description**

The present invention relates to a sensor for monitoring gaseous compounds, particularly to a sensor suitable for indicating the freshness level and/or edibility of a packaged perishable product.

Conventional indicators of the quality of packaged perishable products are mainly found in the form of labels affixed to the outside of the packaging or associated with barcodes. Typically, they involve reactions (chemical or enzymatic) whose rate is dependent of temperature and the substrate (or product) of this reaction is colored. The appearance or disappearance of this color and/or intensity of the coloration thus give information about the time/temperature history that the packaged product may have undergone, and therefore about the roughly anticipated quality evolution of the packaged product. The basic principle is that the final quality is the result of an average initial quality deteriorated through an average degradation rate that is a function of temperature. Their main limitation is that both initial quality and degradation rate are subjected to a high uncertainty.

These indicators are not providing direct information of the quality of the packaged product itself. Indeed, these indicators are generally not able to identify situations in which others parameters than time and temperature, such as a leak in a modified atmosphere packaging or a poor initial quality of the product, are involved by accelerating dramatically product degradation rate and/or shortening the duration required to reach a non acceptable product degradation threshold (reduced shelf-life). Moreover, the initial quality is a key factor, which cannot be taken into account by these indicators.

In a packaging, the most stringent demands for many kinds of packaged fresh perishable products include a hermetic seal aiming at maintaining favorable headspace composition such as vacuum or modified atmosphere (for example low oxygen content, which is a key reactive for most food degradation phenomena, or high carbon dioxide as microbial inhibitor) and sufficiently low storage temperature of the packaging (to slow down degradation rate). If the sealing of an hermetic packaging becomes defective, protective gas (such as carbon dioxide) of a gas-filled packaging leaks out, oxygen and/or humidity, which are deleterious agents as to the extended acceptable quality of most products, can enter the packaging, whereby the prolonged product shelf-life time gained by the initial protective gas filling and low temperature storage is lost. In addition to the product quality preservation technologies, the use of a high quality of the product is an essential factor conditioning food shelf-life.

With the spoilage and/or the maturation of a perishable product, the physical-chemical reactions such as oxidation, enzymatic reaction and microbial activity, releases a large number of compounds, which are affecting or not the sensory acceptability of the product. These compounds are good direct quality indicators of the overall quality of a product. The composition of the compounds formed in spoilage and/or maturation depends on the type of the product. In perishable food products, these compounds may be volatile substances such as ethanol (marker of fermentative degradation and also used as protective vapors against molds development), diacetyl, organic acids, amino compounds, or gaseous substances such as carbon dioxide (marker of many microbial contamination as well as metabolism disorder of fresh fruits and vegetables, and also used as a protective gas to inhibit growth of microorganisms responsible for food spoilage), ammonia, hydrogen sulfide, sulfur dioxide, and gaseous amines.

In order to have appropriate information on product quality and acceptability, it is thus essential to easily monitor the composition of the packaging headspace gaseous compounds either responsible for the degradation of products (water vapor, carbon dioxide, oxygen) or being indicators of product degradation (ethanol, carbon dioxide ..), such as microorganism growth.

It is also desirable to monitor packaging headspace in order to detect the leakage of a protective gas, such as carbon dioxide.

In this respect, WO 03/044521 discloses spoilage sensor for packaged food and drugs, which may advantageously be placed inside the packaging and thus enables a direct control of the quality of the packaged product. However, this sensor is merely sensitive to oxygen and hydrogen sulfide content inside the packaging, which is not sufficient to thoroughly monitor the quality of a packaged perishable product. Notably, this sensor is not suitable for example for monitoring carbon dioxide either as food degradation markers or as a protective gas.

Besides, the method for preparing said sensor involves significant amount of expensive and non eco-friendly materials, such as metals and metallic compounds. Such metals and metallic compounds are not biodegradable and remain in the environment for extended periods of time after the sensors are removed from service and discarded. Of course, some of these materials can be recycled and reused. However, recycling also requires expenditure of natural resources, and adds to the environmental cost associated with such materials. Correspondingly, such sensors are usually discarded and remain in the environment for extended period of time, negatively impacting the environment.

Therefore, there is a need for novel sustainable methods for detecting markers of the spoilage of perishable products by monitoring gaseous substances representing either product degradation markers or protective gaseous headspace atmosphere.

It is an object of the present invention to provide a novel method for monitoring the presence of a gaseous compound in a predetermined volume and/or the variation of the concentration of a gaseous compound in a predetermined volume, said method being advantageously suitable for monitoring the variation of humidity and/or carbon dioxide content.

It is also an object of the present invention to provide a method for indicating the freshness level and/or edibility of packaged perishable products, such as foodstuffs, cosmetics and pharmaceuticals in particular.

It is also an object of the present invention to provide a novel sensor for indicating the freshness level and/or edibility of a packaged perishable product, remotely readable by RF techniques.

The inventors have found that a biopolymer may be used as a sensitive material in a dielectric gas sensor.

A "dielectric gas sensor" is a sensor able to translate into an electric signal the presence and/or the amount of at least one gaseous compound.

One object of the present invention is thus the use of a biopolymer as a sensitive material in a dielectric gas sensor for water vapor, carbon dioxide, and/or ethanol vapor.

One advantage of the present invention is in providing dielectric gas sensors that are biodegradable and minimize the negative impact on the environment.

Another advantage of the present invention is in providing dielectric gas sensors that are biocompatible.

Yet another advantage of the present invention is in providing dielectric gas sensors that have additional functional features that are not provided by conventional sensors, such as detection of various types of gaseous compounds generated by the spoilage of perishable products, and/or detection of a leakage in the protective atmosphere.

The biopolymers suitable for the invention are biopolymers having electrical and/or dielectric properties, which vary according to the gaseous composition of the atmosphere to which they are in contact.

A biopolymer particularly suitable for implementing the present invention is a biopolymer whose at least one electrical or dielectric parameter varies according to the concentration in water vapor, in carbon dioxide, and/or in ethanol vapor in the gaseous composition to which it is in contact, preferably in which it is placed.

The interpretation of the electrical and/or dielectric parameter of these biopolymers allows the detection of a modification of the concentration of a gaseous compound.

By "electrical parameter", it is meant more particularly the capacitance, the impedance or the shift frequency.

By "dielectric parameter", it is meant more particularly the permittivity or the dielectric loss.

"Biopolymer" refers to a polymer of natural origin, such as of animal or vegetal origin.

A biopolymer suitable for the invention preferably comprises at least one protein, such as a vegetal or animal protein, preferentially a vegetal protein.

The electric properties or dielectric properties of a biopolymer are generally due to the presence of electrical charges within its structure.

As it is shown in the examples described further, it was observed that the electrical or dielectric properties of a biopolymer may be modified by a modification of the gaseous composition in contact with said biopolymer, notably by a variation of the concentration in water vapor, carbon dioxide, and/or ethanol vapor.

Without being bound to a particular theory, the absorption of a gaseous compound onto a biopolymer may change the number of electrical charges within the structure of said biopolymer, and/or modify the structure of said biopolymer, thus resulting in a modification of one of its electrical or dielectric parameters. By this way, a biopolymer allows to detect the increase of concentration of a gaseous compound in a predetermined volume.

Similarly, the desorption of a gaseous compound from a biopolymer may change the number of electrical charges within the structure of said biopolymer, and/or modify the structure of said biopolymer, thus resulting in a modification of one of its electrical or dielectric parameter. By this way, a biopolymer allows to detect the decrease of concentration of a gaseous compound in a predetermined volume.

A biopolymer thus may work in a dielectric gas sensor as a sensitive material for gaseous compounds selected form the group consisting of water vapor, carbon dioxide, and ethanol.

Suitable biopolymers for implementing the invention are notably wheat gluten, gelatin and casein.

According to a preferred embodiment, the biopolymer is wheat gluten.

Notably, the inventors have found that wheat gluten is particularly efficient as a sensitive material in a dielectric gas sensor for water vapor, carbon dioxide, and/or ethanol vapor.

"Wheat gluten" refers to a complex mixture of gluten proteins (from 75% to 80% by weight), starch (from 15% to 20% by weight), and fat (from 5% to 7% by weight), found in wheat endosperm.

Gluten proteins include protein subunits gliadins and glutenins, and may include protein hydrolysates and in particular wheat protein hydrolysates.

The gluten proteins are especially characterized by their specific amino acid composition, the protein containing a high content of proline residues (about 30% of the total amino acid residues), and glutamine residues (about 40% of total residues amino acids). The component of gluten proteins are also characterized by the presence of repeated amino acid sequences.

Four classes of gliadin components of gluten are known, respectively, $\alpha$ gliadins, $\beta$ gliadins, $\gamma$ gliadins, and $\omega$ gliadins ($\omega$2 and $\omega$5).

Two classes of glutenin are known, respectively low molecular weight glutenins and high molecular weight glutenins. Low molecular weight glutenins have a molecular weight below 90 000 and the high molecular weight glutenins have a molecular weight equal to or greater than 90 000.

Generally, gluten proteins comprise from 60% to 70% by weight of gliadins and from 30% to 40% by weight glutenins, relative to the total weight of gluten. Gliadins comprise from 4% to 11% by weight of $\omega 5$ gliadins, from 8% to 22% by weight of $\omega 2$ gliadins, from 26% to 29% by weight of $\alpha/\beta$ gliadins, and from 10% to 26% by weight of $\gamma$ gliadins, relative to the total weight of gluten.

According to the present invention, a biopolymer used as a sensitive material is preferably in the form of a coating in contact with at least a part of at least one electrode, said electrode being comprised in the dielectric gas sensor.

By "in contact with", it is meant that the biopolymer adheres to the electrode and that an electric current can traverse the interface biopolymer/electrode.

The biopolymer may be deposited on at least one electrode or at least one electrode may be deposited on a coating of biopolymer. The biopolymer may also be in sandwich between at least two electrodes.

Advantageously, the biopolymer forms a film, having a thickness comprised from $0.1~\mu m$ to $500~\mu m$, in contact with at least a part of at least one electrode, said film being intended to be in contact with the gaseous composition to be analyzed. Accordingly, a biopolymer in contact with said gaseous composition is thus able to interact with the gaseous compounds in said gaseous composition.

The coating is preferably obtained by coating at least a part of at least one electrode with an aqueous solution of said biopolymer and by drying said coating.

The coating preferably comprises more than 50% by weight, preferentially more than 75% by weight, advantageously more than 85% by weight of biopolymer.

According to one embodiment, a dielectric gas sensor for implementing the invention comprises at least one electrode, preferably a plurality of electrodes, said electrodes being at least partly in contact with the biopolymer.

Preferably, a dielectric gas sensor for implementing the invention also comprises a printed circuit board, onto which are deposited the electrode(s).

According to a preferred variant of the invention, a dielectric gas sensor for implementing the invention comprises an interdigital capacitor at least partly coated by a coating comprising the biopolymer, said coating being advantageously in the form of a film as defined above.

An interdigital capacitor comprises a plurality of electrodes, arranged according to a comb-like pattern on a printed circuit board. In said variant of the invention, the comb-like electrodes of the interdigital capacitor are preferably totally coated by the biopolymer and the spaces between these electrodes are also totally coated by the biopolymer.

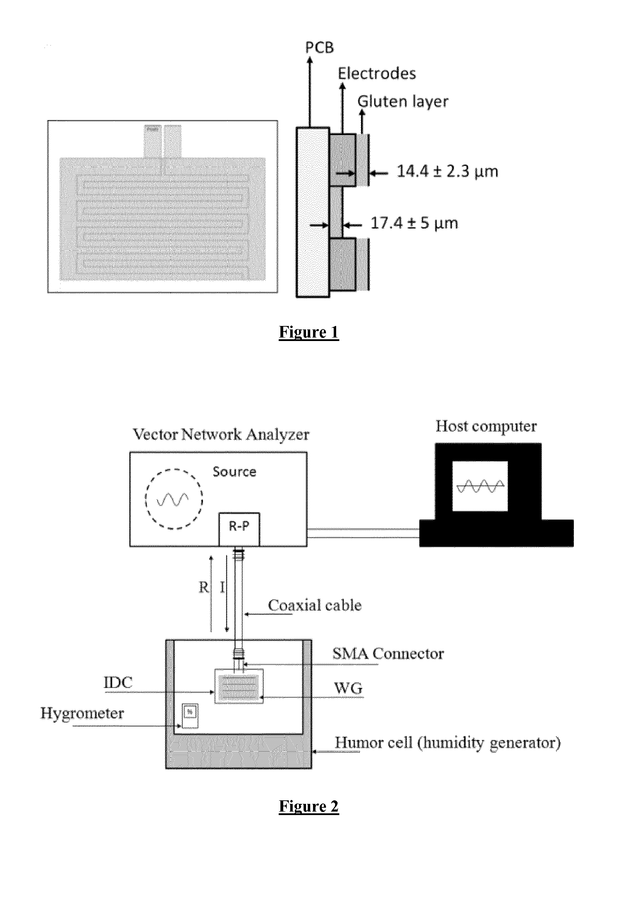

A dielectric gas sensor according to this variant is schematically represented in Figure 1 (wherein PCB means Printed Circuit Board).

Another aspect of the present invention relates to a method for analyzing a gaseous composition using a biopolymer according to the invention as sensitive material.

Another object of the present invention is a method for analyzing the presence and/or the content of water vapor, carbon dioxide and/or ethanol vapor in a gaseous composition, comprising the following steps:

    a. having a biopolymer in contact with said gaseous composition,
    b. measuring at least one electrical or dielectric parameter through said biopolymer, and
    c. evaluating the presence and/or the content of water vapor, carbon dioxide and/or ethanol vapor in said gaseous composition by interpreting the measure carried out in step b, optionally by comparison with a reference value.

In this method, the biopolymer as described above works as a sensitive material as described above, which directly interacts with the gaseous compounds of the gaseous composition to be analyzed. The method of the invention is able to detect the presence and/or to measure the content of water vapor, carbon dioxide and/or ethanol vapor in a gaseous composition.

A biopolymer particularly suitable for implementing the method of the invention is a biopolymer whose at least one electrical or dielectric parameter varies according to the concentration in water vapor, in carbon dioxide, and/or in ethanol vapor in the gaseous composition to which it is in contact, preferably in which it is placed.

According to one embodiment, in step a, the biopolymer is inside the gaseous composition to be analyzed.

According to one embodiment, in step a, the biopolymer is in a dielectric gas sensor comprising at least one electrode, advantageously in the form of a coating in contact with at least a part of said electrode.

Preferably, in step a, the biopolymer is in a dielectric gas sensor as defined above.

Said dielectric gas sensor is preferably placed inside the gaseous composition to be analyzed.

Said coating may be prepared as described above and may have the same characteristics.

According to one embodiment, in step a, the biopolymer is in a dielectric gas sensor in the form of a coating in contact with at least a part of an interdigital capacitor, preferably in the form of a coating on the electrodes of said interdigital capacitor.

According to a first variant, in step a, the biopolymer is in a dielectric gas sensor comprising a RFID tag, so that said sensor is readable remotely by RF techniques.

A RFID tag generally comprises a RFID silicon chip and an antenna, wherein said antenna may be in the form of a planar metal coil.

Preferably, the biopolymer is the form of a coating on the antenna of a RFID tag.

According to another variant, in step a, the biopolymer is in a dielectric gas sensor comprising an electric connector, so that said sensor is readable by connection with an appropriate measurement device.

The biopolymer implemented in the method of the invention may be one of the biopolymers described above.

It was observed that the method of the invention is particularly efficient when wheat gluten is used as biopolymer.

In step b, the measure may be made either directly on the biopolymer using for example a gas sensor having an electric connector, or remotely using for example RF techniques.

The variant wherein the dielectric gas sensor comprises a RFID tag is particularly adapted to the embodiment wherein the measuring step b is made remotely.

The variant wherein the dielectric gas sensor comprises an electric connector is particularly adapted to the embodiment wherein the measuring step b is made directly on the biopolymer.

According to one embodiment, in step b, the electrical parameter which is measured is the capacitance, the impedance or the shift frequency of the biopolymer placed in contact with the gaseous composition to be analyzed.

The measure of the capacitance (i.e. the ability of the biopolymer to store electrical charges) is particularly adapted to the embodiment wherein wheat gluten is used as sensitive biopolymer.

According to another embodiment, in step b, the dielectric parameter which is measured is the permittivity or the dielectric loss of the biopolymer placed in contact with the gaseous composition.

In step c, by interpreting the measure carried out in step b, notably by comparison with a reference value, the skilled person is able to evaluate the presence and/or the content of water vapor, carbon dioxide and/or ethanol vapor in the gaseous composition to be analyzed.

According to a preferred embodiment, the method is implemented with a relative humidity in the gaseous composition to be analyzed of at least 70%, preferentially from 80% to 100%, more preferentially from 80% to 95%.

Relative humidity (abbreviated RH) is the ratio of the partial pressure of water vapor to the equilibrium vapor pressure of water at the same temperature. Relative humidity depends on temperature and the pressure of the system of interest. Relative humidity may be measured by a hygrometer.

The relative humidity may be generated by a perishable product, such as a food product, placed in contact with the gaseous composition to be analyzed.

In the framework of the present invention, the relative humidity is usually considered at a pressure of about 1 atm and at a temperature comprised from 0 °C to 30 °C, preferably from 4 °C to 25 °C.

It was observed that the method of the invention is particularly efficient in these conditions, notably for monitoring the presence and/or for measuring the content of water vapor or carbon dioxide.

Another aspect of the present invention relates to a method for indicating the freshness level and/or edibility of a packaged perishable product, using a readable dielectric gas sensor comprising a biopolymer as sensitive material.

Another object of the present invention is a method for indicating the freshness level and/or edibility of a packaged perishable product, comprising the following steps of:

   a. having a readable dielectric gas sensor in contact with the headspace of the packaging of the perishable product, said dielectric gas sensor comprising at least one electrode at least partly in contact with with a biopolymer,
   b. collecting at least one value of at least one electrical or dielectric parameter through the electrode of said dielectric gas sensor, and
   c. evaluating the freshness level and/or edibility of a packaged perishable product by interpreting the value collected in step b, optionally by comparison with a reference value.

By "perishable product", it is meant a product that may degrade over time, especially when it is not kept in appropriate storage conditions, such as foodstuff, cosmetics or pharmaceutics.

By "headspace", it is meant the space between a packaged perishable product and the corresponding packaging. Said headspace may a gas-filled headspace or a partial vacuum. A gas-filled headspace is notably a headspace filled with a protective gas, such as carbon dioxide or nitrogen. A gas-filled headspace may also be filled with air or with another gas mixture.

A biopolymer particularly suitable for implementing the method of the invention is a biopolymer whose at least one electrical or dielectric parameter varies according to the concentration of at least one marker of the spoilage and/or maturation of the packaged perishable product in the gaseous composition in which it is placed.

Such a biopolymer may be sensitive to a variation of the concentration of several of said markers.

Said markers are for example ethanol, diacetyl, organic acids, amino compounds, water, carbon dioxide, ammonia, hydrogen sulfide, sulfur dioxide, or gaseous amines.

A biopolymer particularly suitable for implementing the method of the invention is a biopolymer whose at least one electrical or dielectric parameter varies according to the concentration in water vapor, in carbon dioxide, and/or in ethanol vapor in the gaseous composition in which it is placed.

According to one embodiment, in step a, the dielectric gas sensor advantageously comprises a biopolymer in the form of a film in contact with at least a part of at least one electrode, or a plurality of electrodes.

According to one embodiment, in step a, the dielectric gas sensor comprises a biopolymer in the form of a coating in contact with at least a part of an interdigital capacitor, preferably in contact with the electrodes of the interdigital capacitor.

According to one embodiment, in step a, the dielectric gas sensor is preferably place inside the headspace of the packaging of the perishable product.

Said coating may be prepared as described above and may have the same characteristics.

According to one embodiment, said dielectric gas sensor may also comprise a RFID tag so that it is readable remotely by RF techniques.

According to said embodiment, the biopolymer is preferably in the form of a film coating at least partly the antenna of the RFID tag.

According to one embodiment, the readable dielectric gas sensor is a RFID readable dielectric gas sensor, which may comprise a passive RFID tag or a semi-passive RFID tag.

According to another embodiment, the readable dielectric gas sensor is a dielectric gas sensor readable using a cable connected to a connector of said dielectric gas sensor.

According to a first variant, the method of the invention implements a readable dielectric gas sensor sensitive to the increase of the concentration of water vapor inside the headspace of the packaged perishable product.

The method according to this embodiment is particularly useful for monitoring the amount of water vapor which is generally generated by the spoilage of a perishable product.

According to another variant, the method of the invention implements a readable dielectric gas sensor sensitive to the decrease of the concentration of carbon dioxide inside the headspace of the packaged perishable product.

The method according to this embodiment is particularly useful for detecting the leakage of carbon dioxide generally used as a protective gas for preventing the spoilage of a perishable product.

According to another variant, the method of the invention implements a readable dielectric gas sensor sensitive to the increase of the concentration of ethanol vapor inside the headspace of the packaged perishable product.

The method according to this embodiment is particularly useful for monitoring the amount of ethanol vapor which is generally generated by the spoilage of a perishable product.

The biopolymer implemented in the method of the invention may be one of the biopolymers described above.

It was observed that the method of the invention is particularly efficient when wheat gluten is used as biopolymer.

In step b, the dielectric gas sensor is read in order to collect at least one value of at least one electrical or dielectric parameter. This value is collected through the electrode coated by the biopolymer, in contact with the gaseous composition in the headspace.

According to one embodiment, in step b, the electrical parameter which is measured is the capacitance, the impedance or the shift frequency of the biopolymer placed in contact with the gaseous composition to be analyzed.

According to another embodiment, in step b, the dielectric parameter which is measured is the permittivity or the dielectric loss of the biopolymer placed in contact with the gaseous composition.

Step b is preferably carried out remotely, for example using RF techniques.

The embodiment wherein the dielectric gas sensor comprises a RFID tag is particularly adapted to the embodiment wherein the collecting step b is made remotely.

In the embodiment wherein the dielectric gas sensor is RFID-readable, the collecting may be carried out at low frequency (such as at a frequency lower than 100 MHz) or at high frequency such as in the RFID UHF range, notably from 1 MHz to 1 GHz, preferably from 860 MHz to 960 MHz, and preferentially at 868 MHz.

In the embodiment wherein the dielectric gas sensor is RFID-readable, the collecting may be carried out with a Tagformance measurement system commercialized by VOYANTIC.

Said system is a widely used tool for verifying tag design, selecting tags for a specific use case, and for evaluating the tag performance in real-life applications. Tagformance is a complete measurement solution, notably for evaluating the functionality and performance of EPC Class 1 Gen2 RFID systems, which are RFID tags suitable for the present invention.

The skilled person in the art is able to use said system for implementing the method of the invention.

In the method of the invention, the biopolymer works as a sensitive material as described above, which directly interacts with the gaseous compounds generated by the spoilage of the perishable product, or which enter/leave the headspace of the packaging.

The readable sensor thus translates into an electrical signal readable by an user the generation of a gaseous compound caused by the spoilage of a perishable product. The readable sensor also translates into a signal readable by an user the increase or decrease of gaseous compound content due to a leak or break in the packaging.

In step c, the user interprets the value collected in step b, optionally by comparison with a reference value, which usually corresponds to a value wherein the packaged perishable product is in good usability conditions. Through step c, the user is thus able to determine whether the packaged perishable product is still in good usability condition.

The method of the invention notably indicates the microbiological decay of a perishable product and/or the degradation of the packaging in a direct and reliable fashion.

The method of the invention allows a control of the packaging headspace in two ways, primarily by monitoring of the packaging integrity, since the concentration of a gaseous compound would decrease consequently if holes are present in the packaging, and secondly by monitoring the shelf-life or maturation of perishable products, since the concentration of a gaseous compound generated by microbial growth would increase inside the packaging.

The method of the invention is particularly useful for detecting the increase of water vapor or ethanol vapor in the headspace packaging of a packaged perishable product.

The method of the invention is also particularly useful for detecting the leakage of carbon dioxide from the carbon dioxide-filled headspace packaging of a packaged perishable product.

The method of the invention thus allows direct and easy monitoring of the quality of a packaged perishable product.

Preferably, the relative humidity inside the headspace of the packaged perishable product is of at least 70%, preferentially from 80% to 95%.

The relative humidity may be generated by a perishable product, such as a food product, placed in contact with the gaseous composition to be analyzed.

Another object of the present invention is a dielectric gas sensor for indicating the freshness level and/or edibility of a packaged perishable product, remotely readable by RF techniques, said sensor comprising at least one electrode at least partly coated with a composition comprising a biopolymer, and a RFID tag.

According to one embodiment, the biopolymer is preferably in the form of a film coating at least partly the antenna of the RFID tag.

The sensor of the invention is intended to placed inside a product packaging, in contact with the headspace of said packaging, for remotely indicating the quality of a perishable packaged product, by the means of RF techniques, known by the skilled person.

The sensor of the invention can be promoted in quality control from a production plant via a transportation chain to the warehousing and retail steps.

Based this technique, the invention provides a disposable spoilage sensor that can be placed in a product packaging so as to be remotely readable without opening/touching the packaging. The remote read technique makes it possible to generate an unambiguous "Accept/Reject" signal. Additionally, the sensor according to the invention allows the condition of a packaged perishable product to be checked, e. g., individually identifiably by unit or case in a production plant, warehouse and/or retail shop without touching the packagings. In a retail shop, an individual packaging can be checked by means of a remote reader device incorporated with a chilled display cabinet or cash register counter. The sensor according to the invention also allows the quality of a gas-filled packaging to be checked.

The sensor of the invention establishes interactions with volatile markers of the quality of the packaged product which affect the parameters of the sensor such as water vapor and ethanol vapor, or with protective gases, such as carbon dioxide, and give information about the actual quality of the packaged product at the time of the reading.

The sensor of the invention is made from renewable resources. Furthermore, since biopolymers are low-cost, it should not greatly impact the final cost of the sensor.

A sensor according to the invention may be produced by a method comprising the steps of:

- providing a biopolymer,
- preparing an aqueous solution of biopolymer to yield an aqueous solution of said biopolymer,
- providing a RFID tag, and
- casting the aqueous solution on at least a part of the RFID tag, preferably the antenna of said RFID tag, and drying the aqueous solution to form a biopolymer film on the RFID tag.

The invention may be illustrated by the attached figures, which represents the variation of electrical and dielectric parameters of a wheat gluten layer deposited on an interdigital capacitor, in various conditions of gaseous compound concentration.

Figure 1 schematically represents a dielectric gas sensor according to the invention, implementing a thin layer of wheat gluten onto an interdigital capacitor.

Figure 2 schematically represents the setup used in the examples for electrical property measurements in controlled conditions of humidity, wherein WG represents a layer of wheat gluten coated onto an interdigital capacitor (IDC), R-P represents the reflection port, I represents the incident signal and R represents the reflected signal.

Figure 3 represents the variation of impedance (in ohm) according to frequency (in MHz), in various conditions of humidity.

Figure 4 represents the variation of capacitance (in nF) according to relative humidity (%).

Figure 5 represents the variation of shift in frequency (in MHz) according to relative humidity (%).

Figure 6 represents the variation of impedance (in ohm) according to frequency (in MHz), in various conditions of carbon dioxide concentration.

Figure 7 represents the variation of capacitance (in pF) according to carbon dioxide concentration (%vol).

Figure 8 represents the variation of impedance (in ohm) according to frequency (in MHz), in various conditions of ethanol concentration.

Figure 9 represents the variation of capacitance (in pF) according to ethanol concentration.

Measurements were made at high frequency (868 MHz):

Figure 10 represents the variation of capacitance (in nF) according to relative humidity (%).

Figure 11 represents the variation of capacitance (in pF) according to carbon dioxide concentration (%vol).

The invention will now be illustrated by the non-limiting examples that follow.

## Examples

### Example 1 - Wheat gluten solution preparation

Wheat gluten (amygluten 110) powder was provided by Amylum (Mesnil St Nicaise, France). Sodium sulfite and acetic acid were used to prepare the coating solution and for pH adjustment respectively. They were purchased from Sigma-Aldrich (St Quentin, France).

A wheat gluten solution was prepared at room temperature and room humidity. First, 30 g of wheat gluten powder were dispersed under shaking in 50 ml of a sodium sulphite solution (0.06 g/50 mL). The mixture was left to settle for 30 minutes. After the allocated time, the pH of the solution was adjusted to 4 by adding 3.4 mL of 50/50 v/v solution of acetic acid. The whole mixture was stirred. The solution was finally adjusted to 130 ml by adding deionised water and mixed. The prepared wheat gluten solution was left to degas under vacuum for one night.

### Example 2 - Sample preparation - Solvent Casting method

The wheat gluten layer was manufactured on interdigital capacitors (IDC) systems using an E409 blade coater from Erichsen (France). The coater was equipped with the n°4 blade having spires of 0.51 mm, in order to create a humid film deposit having a thickness of 40 $\mu$m. The speed was set to 1 mm/s. 1 mL of solution prepared in Example 1 was necessary for the deposit. The sample was left to dry for 24 h at room temperature and at 50% of relative humidity.

### Example 3 - Sensing ability of the wheat gluten film at low frequency (lower than 100 MHz)

The sensing ability of wheat gluten towards vapors and gases was studied at low frequency, suitable for an application in semi-passive RFID tag.

Effects of humidity

Wheat gluten layer deposited on IDCs has been exposed to controlled conditions of humidity in a humidity regulated cell. After a period of stabilization of at least 2 hours, electrical measurements have been performed on the wheat gluten layer.

*Material and Methods*

A coaxial cable, a Vector Network Analyzer (VNA), a humidity generator cell (Humor 10, Feuchte-generator), a hygrometer (Rotronic Hygromer) and a host computer were used for the electrical measurements.

Impedance measurements were performed using a VNA (Hewlett-Packard 8753D) with an open-ended coaxial cable, connected to the IDC system through a coaxial connector (SMA connector). The whole system, composed of the VNA, the coaxial cable and SMA connectors (SubMiniature version A), was calibrated using three different types of loads: open circuit, short circuit and a 50 load to rigorously characterize the electrical properties. The calibration was performed over a frequency range of 30 MHz to 3 GHz.

The coaxial cable was fixed to the extent possible in order to avoid stray capacitors due to cable movements after calibration, before connecting the IDC system. The latter was inserted in the humor cell where relative humidity was controlled, and a hygrometer was used to verify the humidity value. The relative humidity range varied from 20% to 95% and the humidity deviation was ($\pm$1%) at high humidity values. The temperature was maintained constant at 25°C during the whole experiment.

A host computer was connected to the VNA for data acquisition. The real and imaginary impedance components, as well as the frequency, were acquired at fixed time intervals (30 minutes for each measurement) during the specified stabilization time provided by the dynamic vapor sorption measurements. These electrical properties were recorded (as triplicates) by a program developed on Labview software and data was saved in text files.

The experimental setup is schematically presented in Figure 2

The IDC system is equivalent to a resistance-capacitance (RC) circuit.

The associated real and imaginary parts are represented by Equation 1 and Equation 2 respectively, where both are frequency dependant (Dickey et al., 2002; Lacquet and Swart, 1993).

$$\text{Equation 1} \qquad Z_r = \frac{R_p}{1+(R_p C_p \omega)^2}$$

$$\text{Equation 2} \qquad Z_{img} = -\frac{R_p^2 C_p \omega}{1+(R_p C_p \omega)^2}$$

The identification of the dielectric properties of wheat gluten was performed by retro-simulation using ANSOFT® software. The only modification made to the IDC system design was the addition of the dielectric layer upon the IDC surface, representing as far as possible the physical system. The permittivity and dielectric loss were obtained by comparing the simulated impedance values to those measured on the specified frequency range. Impedance matching is obtained by the refinement of several parameters in the software such as the substrate (epoxy resin) and material dielectric permittivity and loss. Consequently the appropriate permittivity and dielectric loss values are obtained after refinement.

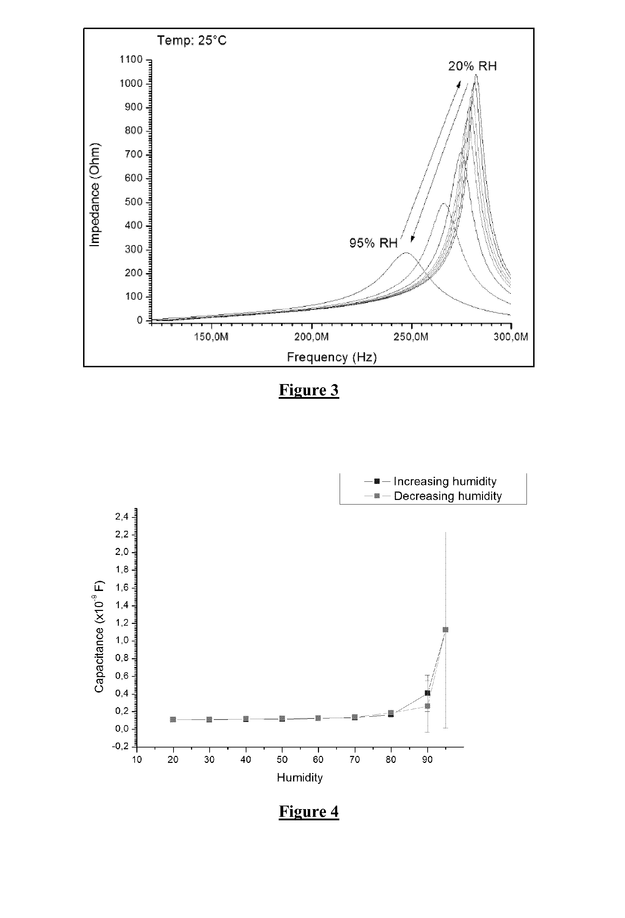

Figure 3 represents the variation of impedance (in ohm) as a function of frequency (in MHz), in various conditions of humidity.

Figure 4 represents the variation of capacitance (in nF) as a function of relative humidity (%).

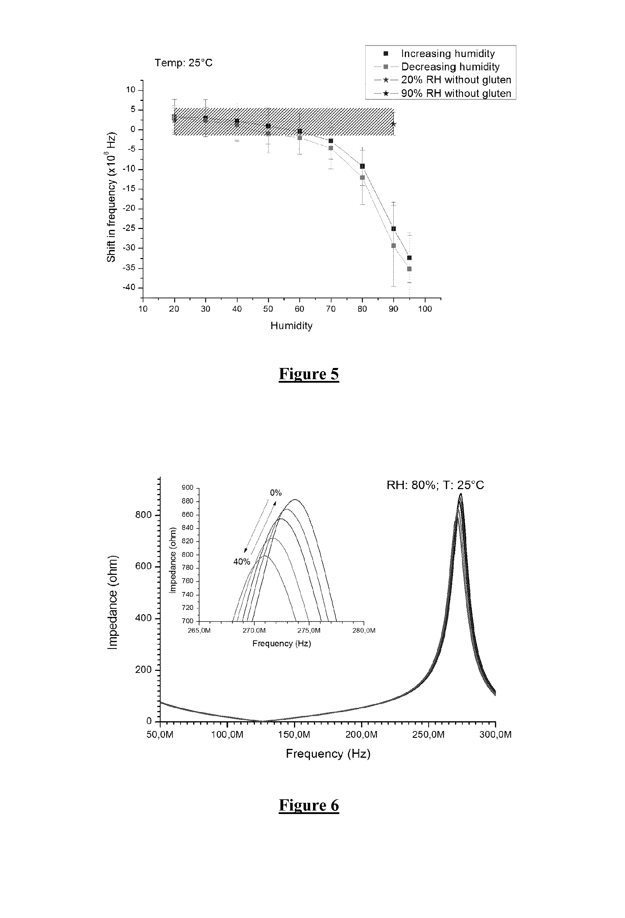

Figure 5 represents the variation of shift in frequency (in MHz) as a function of relative humidity (%).

These results showed that humidity has a reversible effect on the electrical parameters of wheat gluten layer, with a hysteresis. A noticeable effect on the impedance and capacitance value of the wheat gluten layer was observed. This effect is even more pronounced for RH from 70% to 95%.

Effects of carbon dioxide

Wheat gluten layer deposited on IDCs has been exposed to a gas flow having a controlled concentration in carbon dioxide in a regulated cell placed at 80%RH. The carbon dioxide concentration in the gas flow was increased from 0% v/v up to 40% v/v with a 10% step. After a period of stabilization of 12 hours, electrical measurements have been performed on the wheat gluten layer, using the same method as described above.

Figure 6 represents the variation of impedance (in ohm) as a function of frequency (in MHz), in various conditions of carbon dioxide concentration.

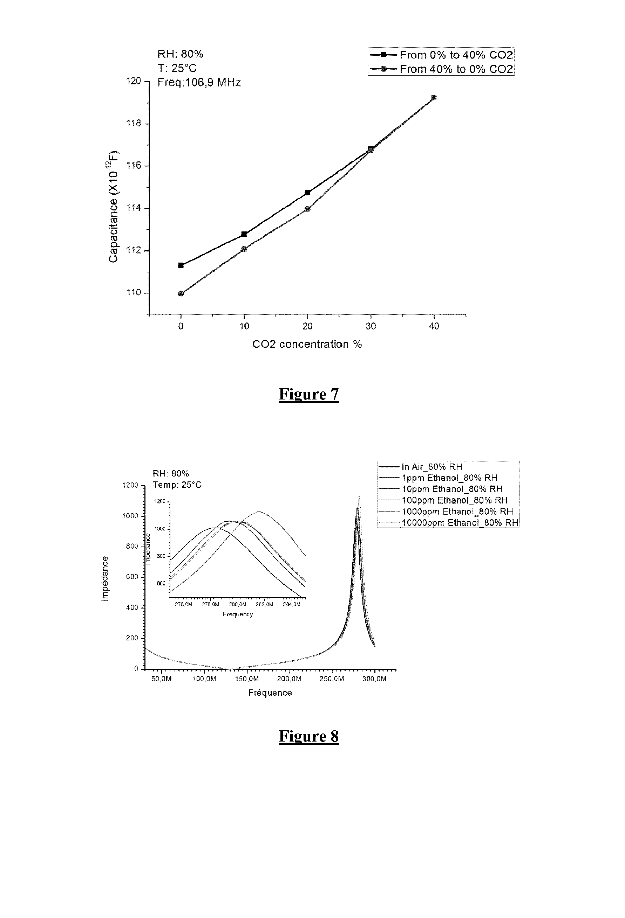

Figure 7 represents the variation of capacitance (in pF) as a function of carbon dioxide concentration (%vol).

These results showed that carbon dioxide has a reversible effect on the electrical parameters of wheat gluten layer. As illustrated by Figure 7, there is a linear and reversible increase in the capacitance value from 111 pF to 119 pF (Figure 7).

Effects of ethanol

Wheat gluten layer deposited on IDCs has been exposed to controlled concentrations of ethanol vapor in a regulated cell. The ethanol concentration was increased from 1 ppm to 10 000 ppm. After a period of stabilization of 12 hours, electrical measurements have been performed on the wheat gluten layer, using the same method as described above.

Figure 8 represents the variation of impedance (in ohm) as a function of frequency (in MHz), in various conditions of ethanol concentration.

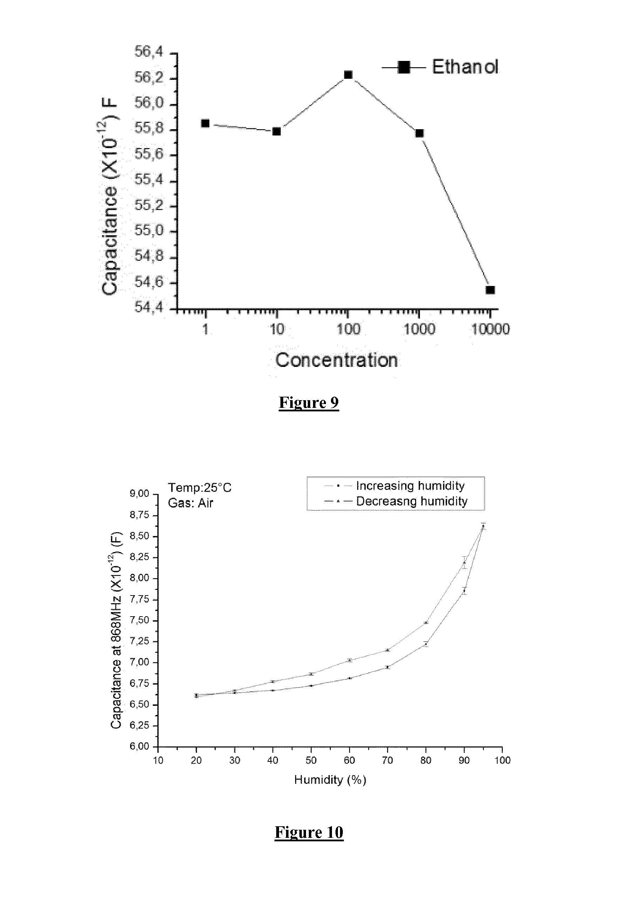

Figure 9 represents the variation of capacitance (in pF) as a function of ethanol concentration.

Conclusion

Humidity was found to induce the greatest change in capacitance profile, mainly above 70% RH. Change in electrical parameters caused by moisture variation from 70 to 95%RH are high and therefore perfectly adapted to detect moisture variations.

Positive results were also demonstrated for carbon dioxide and ethanol.

**Example 4 - Sensing ability of the wheat gluten film at high frequency (868 MHz)**

The sensing ability of wheat gluten towards vapors and gases was studied at high frequency, suitable for an application in passive RFID tag.

Effects of humidity

Wheat gluten layer deposited on IDCs has been exposed to controlled conditions of humidity in a humidity regulated cell. After a period of stabilization of 2 hours, electrical measurements have been performed on the wheat gluten layer, using the same method as described above.

Figure 10 represents the variation of capacitance (in nF) as a function of relative humidity (%).

These results showed that the electrical parameters varied in a large extent with increasing humidity. Indeed, the capacitance increased considerably between 60% and 95% RH. A reversibility of electrical parameters of wheat gluten can also be noticed, with a hysteresis.

Effects of carbon dioxide

Wheat gluten layer deposited on IDCs has been exposed to a gas flow having a controlled concentration in carbon dioxide in a regulated cell placed at 95%RH. The carbon dioxide concentration in the gas flow was increased from 0% v/v up to 40% v/v with a 10%. After a period of stabilization of 12 hours, electrical measurements have been performed on the wheat gluten layer, using the same method as described above.

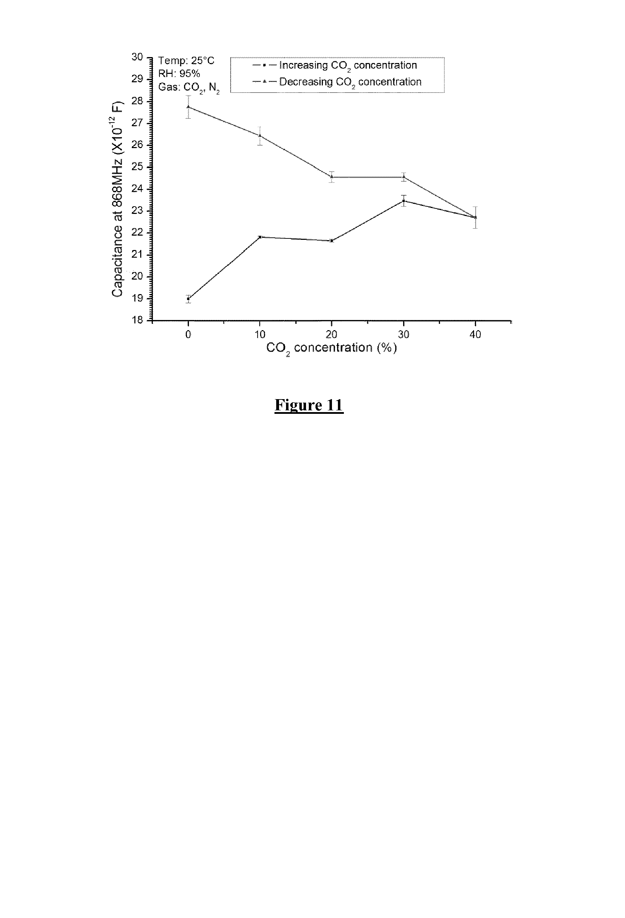

Figure 11 represents the variation of capacitance (in pF) as a function of carbon dioxide concentration (%vol).

A change in electrical parameters was induced at 95%RH with increasing carbon dioxide concentration. It can be found that the process is a non-reversible one.

Conclusion

Effects of humidity and carbon dioxide, but also of others markers such as ethanol, on the electrical parameters of the wheat gluten layer have been demonstrated at 100 MHz and 868 MHz, proving the sensing ability of biopolymers such as wheat gluten. This sensing ability may be used for designing sensor of volatile markers of food degradations such as humidity, carbon dioxide and ethanol.

**Example 5 - Sensing ability of the wheat gluten/RFID tag towards carbon dioxide at high frequency**

The antenna of a RFID tag (EOS 500 DE TAGEOS Cie, MONTPELLIER, FR.) was coated with a wheat gluten solution as prepared in Example 1. An uncoated RFID tag was also used as a reference.

The reference RFID tag was placed inside a regulated cell, and the following successive conditions were set:

    a. 0% carbon dioxide and 90% RH,
    b. 50% carbon dioxide and 90% RH for 60 hours,
    c. 3 hours of dry nitrogen flow, and
    d. 100% carbon dioxide and 90% RH for 16 hours.

The coated RFID tag was placed inside a regulated cell, and the following successive conditions were set:

    a. 0% carbon dioxide and 90% RH,

b. 50% carbon dioxide and 90% RH for 60 hours,

c. 3 hours of dry nitrogen flow,

d. 100% carbon dioxide and 90% RH for 16 hours, and

e. 9 hours of dry nitrogen flow.

At each step, the response of each tag was recorded. The theoretical read range was measured according to the frequency (from 700 MHz to 1200 MHz) using a Tagformance measurement system commercialized by VOYANTIC, with a method known by the skilled person.

Almost no variation in the theoretical read range was observed with the reference RFID tag through the different steps a to d.

By contrast, the wheat gluten coated RFID tag according to the invention exhibited a significant modification of theoretical read range after steps b and d. This result shows that the RFID tag according to the invention is able to detect a modification of relative humidity.

Interestingly, the theoretical read range went back to the initial value after step e, showing that the process is reversible.

## Claims

1. Use of a biopolymer as a sensitive material in a dielectric gas sensor for water vapor, carbon dioxide, and/or ethanol vapor.

2. The use according to claim 1, wherein the biopolymer is a biopolymer whose at least one electrical or dielectric parameter varies according to the concentration in water vapor, in carbon dioxide, and/or in ethanol vapor in the gaseous composition in which it is placed.

3. The use according to claim 1 or 2, wherein the biopolymer comprises at least one protein, such as a vegetal or animal protein, preferably a vegetal protein.

4. The use according to claim 3, wherein the biopolymer is wheat gluten.

5. The use according to any one of claims 1 to 4, wherein the biopolymer is in the form of a coating in contact with at least a part of at least one electrode, said electrode being comprised in the dielectric gas sensor.

6. The use according to any one of claims 1 to 5, wherein the dielectric gas sensor comprises an interdigital capacitor at least partly coated by a coating comprising the biopolymer.

7. A method for analyzing the presence and/or the content of water vapor, carbon dioxide and/or ethanol vapor in a gaseous composition, comprising the following steps:

a. having a biopolymer in contact with said gaseous composition,

b. measuring at least one electrical or dielectric parameter through said biopolymer, and

c. evaluating the presence and/or the content of water vapor, carbon dioxide and/or ethanol vapor in said gaseous composition by interpreting the measure carried out in step b, optionally by comparison with a reference value.

8. The method according to claim 7, wherein the biopolymer is a biopolymer whose at least one electrical or dielectric parameter varies according to the concentration in water vapor, in carbon dioxide, and/or in ethanol vapor in the gaseous composition in which it is placed.

9. The method according to claim 7 or 8, wherein the biopolymer is in a dielectric gas sensor, preferably a dielectric gas sensor as defined in any one of claims 1 to 6.

10. The method according to any one of claims 7 to 9, wherein the biopolymer is in the form of a coating in contact with at least a part of at least one electrode, said electrode being comprised in a dielectric gas sensor.

11. The method according to any one of claims 7 to 10, wherein the biopolymer is wheat gluten.

**12.** The method according to any one of claims 7 to 11, wherein the relative humidity in the gaseous composition is of at least 70%, preferentially from 80% to 100%, more preferentially from 80% to 95%.

**13.** A method for indicating the freshness level and/or edibility of a packaged perishable product, comprising the following steps of:

a. having a readable dielectric gas sensor in contact with the headspace of the packaging of the perishable product, said dielectric gas sensor comprising at least one electrode at least partly in contact with a biopolymer,
b. collecting at least one value of at least one electrical or dielectric parameter through the electrode of said dielectric gas sensor, and
c. evaluating the freshness level and/or edibility of a packaged perishable product by interpreting the value collected in step b, optionally by comparison with a reference value.

**14.** The method according to claim 13, wherein the packaged perishable product is a packaged perishable foodstuff.

**15.** The method according to claim 13 or 14, wherein the readable dielectric gas sensor comprises a RFID tag.

**16.** The method according to claim 15, wherein the collecting step is carried out using RF techniques.

**17.** The method according to any one of claims 13 to 16, wherein the biopolymer is a biopolymer whose at least one electrical or dielectric parameter varies according to the concentration in water vapor, in carbon dioxide, and/or in ethanol vapor in the gaseous composition in which it is placed.

**18.** The method according to any one of claims 13 to 17, wherein the biopolymer is wheat gluten.

**19.** A dielectric gas sensor for indicating the freshness level and/or edibility of a packaged perishable product, remotely readable by RF techniques, said sensor comprising an electrode at least partly in contact with a composition comprising a biopolymer, and a RFID tag.

**Figure 1**

**Figure 2**

**Figure 3**

**Figure 4**

**Figure 5**

**Figure 6**

RH: 80%
T: 25°C
Freq:106,9 MHz

From 0% to 40% CO2
From 40% to 0% CO2

Capacitance (X10$^{-12}$F)

CO2 concentration %

**Figure 7**

RH: 80%
Temp: 25°C

In Air_80% RH
1ppm Ethanol_80% RH
10ppm Ethanol_80% RH
100ppm Ethanol_80% RH
1000ppm Ethanol_80% RH
10000ppm Ethanol_80% RH

Impédance

Fréquence

**Figure 8**

**Figure 9**

**Figure 10**

**Figure 11**

Europäisches
Patentamt

European
Patent Office

Office européen
des brevets

# EUROPEAN SEARCH REPORT

Application Number

EP 15 30 6815

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | B. KUMAR ET AL: "Controlled conductive junction gap for chitosan-carbon nanotube quantum resistive vapour sensors", JOURNAL OF MATERIALS CHEMISTRY, vol. 22, no. 21, 1 January 2012 (2012-01-01), page 10656, XP055251906, GB ISSN: 0959-9428, DOI: 10.1039/c2jm30527e | 7-12 | INV. G01N27/22 G01N33/12 B65B25/00 G01N33/02 |
| A | * lines 4-9, left-hand column, page 10657; sections II.3, II.7 and III.4. * | 1-6, 13-19 | |
| X | Erica Nicole Jones: "DEVELOPMENT OF BIOPOLYMER BASED RESONANT SENSORS", , 1 May 2010 (2010-05-01), XP055276468, Retrieved from the Internet: URL:https://etd.ohiolink.edu/rws_etd/docum ent/get/dayton1272992841/inline [retrieved on 2016-05-30] | 1-12 | |
| Y | * "Background" of chapter 2; sections 2.2, 5.2 and 6.2. * | 13-19 | TECHNICAL FIELDS SEARCHED (IPC) |
| Y | RADISLAV A. POTYRAILO ET AL: "Battery-free Radio Frequency Identification (RFID) Sensors for Food Quality and Safety", JOURNAL OF AGRICULTURAL AND FOOD CHEMISTRY, vol. 60, no. 35, 5 September 2012 (2012-09-05), pages 8535-8543, XP055104311, ISSN: 0021-8561, DOI: 10.1021/jf302416y | 13-19 | G01N B65B |
| A | * Page 8536, first full sentence of right-hand column; Section entitled "Monitoring of Fish Condition".; figure 8 * | 1-12 | |

-/--

The present search report has been drawn up for all claims

| Place of search | Date of completion of the search | Examiner |
|---|---|---|
| The Hague | 31 May 2016 | Martin, Hazel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 15 30 6815

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2008/274912 A1 (JOHNSON ALAN T [US] ET AL) 6 November 2008 (2008-11-06) | 7-12 | |
| A | * Summary of the invention; paragraph [0022] - paragraph [0027]; tables 1, 2 * | 1-6, 13-19 | |
| | ----- | | |
| X | US 5 609 096 A (KWON CHUL H [KR] ET AL) 11 March 1997 (1997-03-11) | 7-12 | |
| A | * column 2 - column 5 * | 1-6, 13-19 | |
| | ----- | | |
| X | QI HAISONG ET AL: "Unique water sensors based on carbon nanotube-cellulose compos", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, vol. 185, 6 May 2013 (2013-05-06), pages 225-230, XP028595407, ISSN: 0925-4005, DOI: 10.1016/J.SNB.2013.04.116 | 7-12 | |
| A | * abstract; Introduction; Experimental. * | 1-6, 13-19 | TECHNICAL FIELDS SEARCHED (IPC) |
| | ----- | | |

The present search report has been drawn up for all claims

| Place of search | Date of completion of the search | Examiner |
|---|---|---|
| The Hague | 31 May 2016 | Martin, Hazel |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

   ......................................................................................
& : member of the same patent family, corresponding
   document

EPO FORM 1503 03.82 (P04C01)

Europäisches
Patentamt

European
Patent Office

Office européen
des brevets

Application Number

EP 15 30 6815

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

**Europäisches Patentamt**
**European Patent Office**
**Office européen des brevets**

## LACK OF UNITY OF INVENTION
### SHEET B

Application Number

EP 15 30 6815

---

The Search Division considers that the present European patent application does not comply with the requirements of unity of invention and relates to several inventions or groups of inventions, namely:

1. claims: 1-12

   Method for analysing presence and/or content of water vapour, carbon dioxide and/or ethanol vapour in gaseous composition

   ---

2. claims: 13-19

   Method for indicating the freshness level and/or edibility of a packaged perishable product

   ---

## ANNEX TO THE EUROPEAN SEARCH REPORT
## ON EUROPEAN PATENT APPLICATION NO.

EP 15 30 6815

This annex lists the patent family members relating to the patent documents cited in  the above-mentioned European search report.
The members are as contained in the European Patent Office EDP file on
The European Patent Office is in no way liable for these particulars which are merely given for the purpose of information.

31-05-2016

| Patent document cited in search report | | Publication date | Patent family member(s) | | Publication date |
|---|---|---|---|---|---|
| US 2008274912 | A1 | 06-11-2008 | US | 2008274912 A1 | 06-11-2008 |
| | | | WO | 2006110347 A2 | 19-10-2006 |
| US 5609096 | A | 11-03-1997 | CN | 1115848 A | 31-01-1996 |
| | | | JP | 2875174 B2 | 24-03-1999 |
| | | | JP | H07209279 A | 11-08-1995 |
| | | | US | 5609096 A | 11-03-1997 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03044521 A